(19) Europäisches Patentamt
European Patent Office
Office européen des brevets

(11) **EP 1 841 508 B1**

(12) **FASCICULE DE BREVET EUROPEEN**

(45) Date de publication et mention
de la délivrance du brevet:
**22.02.2017 Bulletin 2017/08**

(21) Numéro de dépôt: **05825973.0**

(22) Date de dépôt: **14.12.2005**

(51) Int Cl.:
*A61Q 19/04* (2006.01)   *A61Q 17/04* (2006.01)
*A61Q 19/08* (2006.01)   *A61K 8/97* (2017.01)
*A61K 8/49* (2006.01)

(86) Numéro de dépôt international:
**PCT/FR2005/003125**

(87) Numéro de publication internationale:
**WO 2006/075059 (20.07.2006 Gazette 2006/29)**

(54) **LYOPHILISAT DE CELLULES D'ALGUES BRUNES, PROCEDE D'OBTENTION ET UTILISATIONS**

LYOPHILISAT VON BRAUNALGENZELLEN UND VERFAHREN ZU SEINER GEWINNUNG

BROWN ALGAE CELL LYOPHILISATE, METHOD FOR THE OBTENTION THEREOF

(84) Etats contractants désignés:
**AT BE BG CH CY CZ DE DK EE ES FI FR GB GR
HU IE IS IT LI LT LU LV MC NL PL PT RO SE SI
SK TR**

(30) Priorité: **14.01.2005 FR 0500389**

(43) Date de publication de la demande:
**10.10.2007 Bulletin 2007/41**

(73) Titulaire: **BiotechMarine
22260 Pontrieux (FR)**

(72) Inventeur: **MEKIDECHE, Nicole
F-22620 Ploubazlanec (FR)**

(74) Mandataire: **Conan, Philippe Claude et al
L'Air Liquide SA
Direction de la Propriété Intellectuelle
75, quai d'Orsay
75321 Paris Cedex 07 (FR)**

(56) Documents cités:
**CH-A- 500 711        FR-A- 2 489 689
FR-A- 2 697 409     FR-A- 2 838 341
FR-A- 2 844 449     US-A1- 2003 017 185**

- **PATENT ABSTRACTS OF JAPAN vol. 2003, no.
12, 5 décembre 2003 (2003-12-05) & JP 2004
075634 A (OGURAYA YAMAMOTO:KK), 11 mars
2004 (2004-03-11)**
- **PATENT ABSTRACTS OF JAPAN vol. 2003, no.
01, 14 janvier 2003 (2003-01-14) & JP 2002 265313
A (KOSE CORP), 18 septembre 2002 (2002-09-18)**

## Description

L'invention concerne un lyophilisat de cellules d'algues brunes, son procédé d'obtention, une composition cosmétique le contenant, un complément alimentaire le contenant et les utilisations de ceux-ci.

Domaine de l'invention

La classe des algues brunes, encore appelées fucophycées ou phéophycées, fait partie de l'embranchement des chromophytes ou "algues colorées" souvent appelées chrysophytes.

Cet embranchement rassemble les algues dont les cellules contiennent des pigments caroténoïdes "surnuméraires", comme la fucoxanthine, en plus des pigments chlorophylliens : chlorophylle a et c.

La classe des algues brunes comprend les ordres des : Ascoseirales, Chordariales, Cutlériales, Desmaréstiales, Dictyosiphonales, Dictyotales, Durvilléales, Ectocarpales, Fucales, Laminariales, Nothéiales, Scytosiphonales, Sphacélariales, Sporochnales, Syringodermatales, Tiloptéridales.

Tous les organismes photosynthétiques utilisent des pigments pour capturer l'énergie de la lumière, habituellement une forme de chrorophylle. La chlorophylle standard est la chlorophylle a et elle est essentielle pour le transfert de l'énergie capturée de la lumière aux molécules qui vont utiliser cette énergie. La plupart des organismes chlorophylliens ont d'autres pigments pour capturer plus de lumière mais l'énergie capturée doit toujours être transférée à une molécule de chlorophylle a.

Les algues brunes utilisent plusieurs tels pigments surnuméraires comme la chlorophylle c et les caroténoïdes. Les phéophycées présentent de grandes quantités de caroténoïdes dans leurs plastides et ce sont ces pigments bruns et jaunes qui leur donnent cette couleur brune caractéristique. Le pigment caroténoïde le plus important chez les algues brunes est la fucoxanthine qui tire son nom du Fucus. La fucoxanthine absorbe les longueurs d'onde de 500 à 580 nm.

Les pigments caroténoïdes possèdent une structure aliphatique ou alicyclique. Ils sont liposolubles, ce qui favorise leur intégration directe dans certaines membranes. De ce fait, leur solubilité dans l'eau ne peut se produire que lorsqu'ils sont liés à d'autres molécules. C'est également pourquoi leur métabolisme est le plus souvent directement lié au métabolisme des lipides.

Les caroténoides sont également appelés pigments accessoires puisqu'ils doivent transférer l'énergie qu'ils capturent à la chlorophylle a. Ces pigments sont connus du grand public par le carotène qui a donné son nom à cette famille de pigments.

Les animaux et l'homme en particulier ont pour seule source de caroténoides leur alimentation. Les caroténoides ont une fonction anti-oxydante dans le plasma humain, notamment vis-à-vis des lipides circulants. Cette fonction s'exprime également au niveau des membranes des cellules où ils sont incorporés.

Les caroténoides ont un rôle direct sur la coloration de la peau (jaune-orangé) imitant le bronzage naturel.

Art antérieur

Le carotène est utilisé en complément alimentaire, à prendre quelques jours avant et pendant l'exposition au soleil, pour accélérer le bronzage. Le beta-carotène est le précurseur de la vitamine A. La prise de beta-carotène retarde l'apparition de l'érythème solaire. La vitamine A augmente la quantité de mélanine produite sous l'effet d'un rayonnement bleu. De plus, la vitamine A a un rôle protecteur anti-oxydant. Les vitamines A et E évitent la dénaturation oxydative de la mélanine sous l'effet des UV.

Le carotène est associé dans les compléments alimentaires connus à la vitamine C, la vitamine E ou des flavonoïdes.

L'inconvénient de l'utilisation du carotène pour colorer la peau est que la couleur obtenue est beaucoup plus orangée que celle d'un bronzage naturel. De plus, il est rapidement dégradé en vitamine A.

L'astaxanthine a été montrée être un puissant anti-oxydant. La demande US 6,433,025 décrit son utilisation par voie orale pour prévenir et retarder les coups de soleil.

La canthaxanthine, autre pigment caroténoïde, a été utilisée en complément alimentaire pour colorer la peau et la protéger des rayons UV (GB 1 323 800). Cependant, cette application de la canthaxanthine est interdite en France pour risque de cécité alors qu'elle est toujours commercialisée aux Etats-Unis (Canthorex, Laboratoires DELTA®).

Le cancer de la peau ou mélanome sera le plus fréquent dans les prochaines années en France et l'est déjà aux USA avec environ 800 000 cas/an. Il est le plus souvent la conséquence d'une prolifération intempestive de cellules de la peau suite à des mutations de l'ADN provoquées par les rayons ultraviolets (UV). Les populations prennent peu à peu conscience de ces risques et veulent se protéger du soleil. Cependant, une peau bronzée reste une caractéristique avantageuse et recherchée répondant aux critères esthétiques actuels.

Les fabricants de préparations cosmétiques et pharmaceutiques sont donc constamment à la recherche de principes actifs pour pigmenter artificiellement la peau et la protéger des effets néfastes des UV.

Résumé de l'invention

La Demanderesse a découvert de manière surprenante et inattendue qu'un lyophilisat de gamétophytes d'algues brunes, enrichi en fucoxanthine permet d'atteindre cette combinaison d'effets recherchée :

il pigmente l'épiderme avec une parfaite innocuité en stimulant la mélanogenèse, même sans irradiation UV, tout en le protégeant par une activité anti-radicalaire et stimulant sa régénération.

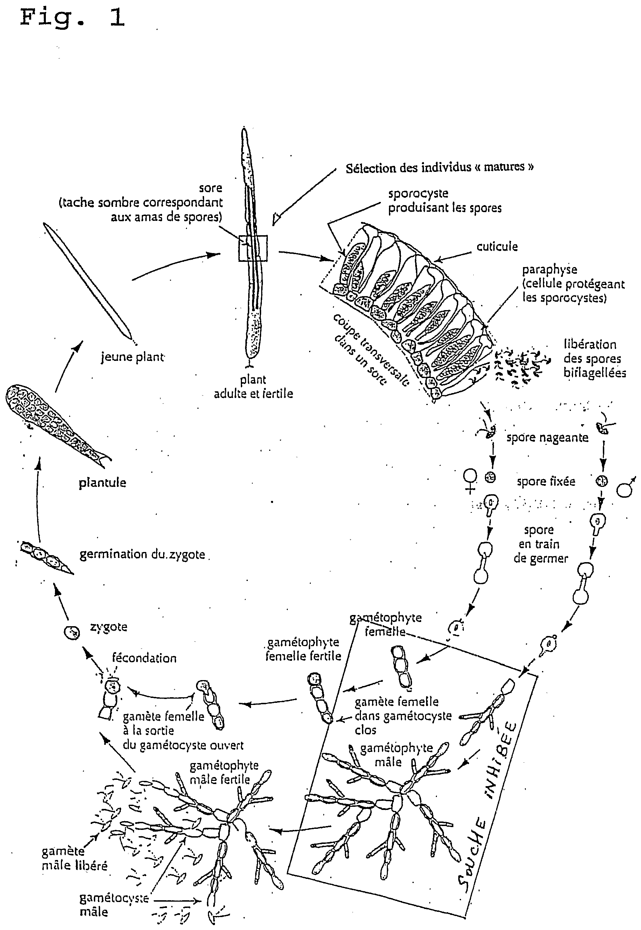

En effet, les algues brunes ont un cycle complexe digénétique. Chez les Laminariales (voir Figure 1), on peut observer l'alternance entre un sporophyte diploïde développé en thalle macroscopique et des gamétophytes mâles et femelles haploïdes microscopiques. Les gamétophytes ne sont dans ce cas qu'un ensemble de cellules issu de différentes mitoses de la spore.

Le gamétophyte est fugace dans le temps et n'est que le stade de production des gamètes. Comme il n'est pas développé en véritable thalle, il fait peu de photosynthèse et renferme un taux maximum de pigments protecteurs : la fucoxanthine, par rapport aux véritables pigments photosynthétiques que sont les chlorophylles.

La Demanderesse a donc sélectionné ce stade gamétophyte pour obtenir un lyophilisat de cellules d'algues brunes extrêmement enrichi en fucoxanthine, facilement et à faible coût par rapport à une extraction de fucoxanthine classique, à partir d'une biomasse disponible à volonté et toute l'année.

Description détaillée de l'invention

L'objet premier de la présente invention est donc un lyophilisat de gamétophytes d'algues brunes enrichi en fucoxanthine, en particulier qui comprend au moins 1 % de fucoxanthine.

Le second objet de la présente invention est un procédé d'obtention d'un lyophilisat de cellules de gamétophytes d'algues brunes qui comprend les étapes suivantes :

- récolte des sporophytes matures
- émission des spores in vitro
- germination des spores in vitro
- récolte des cellules de gamétophytes
- lyophilisation des cellules de gamétophytes obtenues

On appelle "sporophyte mature" un sporophyte qui comprend des spores dans des réceptacles particuliers, par exemple des sores.

Le troisième objet de la présente invention est une préparation cosmétique à usage topique qui comprend un lyophilisat selon l'invention en tant que principe actif, de préférence en une quantité se trouvant dans la gamme de 0,2 à 5% en poids, de manière particulièrement préférée, de 1 à 2% en poids.

Le quatrième objet de la présente invention est un complément alimentaire qui comprend un lyophilisat selon l'invention en tant que principe actif.

La préparation à usage topique comprenant un lyophilisat selon l'invention en tant que principe actif, peut être utilisée pour pigmenter la peau, pour préparer la peau à une exposition aux rayons UV, pour protéger la peau du stress oxydatif provoqué par les rayons UV et/ou pour protéger la peau contre le vieillissement cellulaire.

Le complément alimentaire selon l'invention peut être utilisé pour préparer la peau à une exposition aux rayons UV.

Légende des Figures

Figure 1 : cycle de reproduction des Laminariales
Figure 2 : coupes d'épidermes traité/non traité colorés à l'HES (hématoxyline/éosine/safran)

Exemple 1: Obtention de gamétophytes d'algues brunes laminariales

Des cellules de gamétophytes ont été obtenues pour les espèces suivantes : Laminaria saccharina, Laminaria hyperborea, Alaria esculenta, Undaria pinnatifida.

On récolte des thalles sporophytes fertiles matures présentant des sores (voir Figure 1) à JO sur les côtes françaises bretonnes entre octobre et février.

On les nettoie à l'eau de mer filtrée et on les découpe en morceaux de 5 à 10 cm2 que l'on trempe successivement dans de l'eau de mer filtrée, dans de l'eau de javel à 0,2% pendant 20 à 30 secondes, dans deux bacs d'eau de mer filtrée.

Ensuite, on sèche les morceaux de thalle sur du papier absorbant, on les répartit par 30 sur une feuille de papier absorbant et on roule les feuilles de papier absorbant.

Les feuilles de papier absorbant roulées renfermant les morceaux de thalles sont incubées pendant 12 h à 15°C.

Ensuite, on les place dans de l'eau de mer filtrée et les spores sont alors émises.

On incube les spores dans un milieu de culture défini contenant 0,1% de solution de Provasoli (voir Tableau 1) dans un ballon en présence de lumière (1800-2000 lux, 24h/24) sous agitation douce.

La température est augmentée d'un demi degré par jour jusqu'à 22°C. Le milieu est renouvelé tous les 15 jours.

Les spores germent au bout de 6 à 20 jours de culture et les gamétophytes sont récoltés au bout de 10-12 jours après la germination (voir Tableau 2).

Tableau 1 : composition de la solution de Provasoli pour 1L

| EDTA | 3 g |
|---|---|
| Fe (Cl) | 0,08 g |
| Mn (Cl) | 0,12 g |
| Zn (Cl) | 0,015 g |
| Co (Cl) | 0,003 g |
| Ca (So$_4$) | 0,0012 g |
| B (H$_3$BO$_3$) | 0,6 g |
| Mo (Na$_2$MoO$_4$) | 0,05 g |

Tableau 2

| Espèce | Temps de germination en jours | Obtention des gamétophytes à |
|---|---|---|
| Laminaria saccharina | 9 | J19-J21 |
| Laminaria hyperborea | 11-20 | J21-J32 |
| Alaria esculenta | 6-7 | J16-J19 |
| Undaria pinnatifida | 7 | J17-J19 |

Exemple 2 : Obtention d'un lyophilisat de cellules de gamétophytes d'algues brunes

Inhibition du gamétophyte par un milieu dépourvu en sulfate de kanamycine et dioxyde de germanium pour empêcher la germination de la plantule.

On filtre la culture sur tamis, on rince les cellules à l'eau de mer et on les lyophilise dans un lyophilisateur à plaques.

Pour titrer la fucoxanthine par HPLC, on fait une extraction à l'éthanol à raison de 0,05% de produit lyophilisé pour 10 ml d'éthanol à 70%, agitation 6 h à l'obscurité puis filtration.

L'analyse par HPLC se fait sur colonne Adsorbsphere (Alltech) avec pour solvant/gradient :

A = Acétate d'ammonium/méthanol (20:80)
B = Acétonitrile à 90%
C = Acétate d'éthyle

Exemple 3 : Préparation du produit BB

On prépare selon l'Exemple 2 un lyophilisat de gamétophytes de l'algue Undaria pinnatifida (Laminariacées).

Afin d'utiliser ce lyophilisat de façon appropriée sur culture de peau, le lyophilisat est remis dans le milieu de culture des gamétophytes). Ce mélange est sonifié puis filtré. Le résultat de ce produit filtré est appelé produit « BB ».

Exemple 4 : Evaluation de la tolérance muqueuse d'un lyophilisat de cellules de gamétophytes d'algues brunes par étude de la cytotoxicité sur cornée reconstituée SKINETHIC®

1- Protocole expérimental

On utilise des kératinocytes de la lignée TR 146, transformée spontanément amplifiée par culture cellulaire en milieu défini MCDB 153 modifié.

Lorsqu'ils sont cultivés à l'interface air/liquide en milieu défini, ces kératinocytes humains forment un épithélium pluristratifié sans couche cornée ressemblant à la cornée humaine.

On applique le produit à étudier, ici le produit BB selon l'Exemple 3, à raison de 30 µL sur la surface de huit cultures équivalentes à l'aide d'une micro-pipette. Ces cultures sont ensuite incubées à 37°C, 5% de $CO_2$, pendant 10 minutes, 1 heure, 3 heures et 24 heures, à raison de deux cultures par temps d'incubation.

Les contrôles négatifs (solution saline tamponnée) et positifs (SDS 0,5% et 1%) sont préparés stérilement et déposés de la même manière sur deux cultures, respectivement.

Ces cultures sont ensuite incubées à 37°C, 5% de $CO_2$, pendant 1 heure et 24 heures, à raison de deux cultures par temps d'incubation.

2- Evaluation de la viabilité cellulaire

La viabilité cellulaire est ensuite mesurée qualitativement après marquage par un colorant vital. Le système MTT mesure l'activité déshydrogénase mitochondriale des cellules vivantes. La composante clef est le bromure de 3-[4,5-diméthylthiazol-2-yl]-2,5-diphényl tétrazolium ou MTT.

Les solutions salines tamponnées de MTT, en absence de rouge de phénol, sont de couleur jaune. Les déshydrogénases mitochondriales des cellules vivantes coupent le cycle tétrazolium, induisant ainsi la formation de cristaux pourpres de formazan, insolubles dans les solutions aqueuses.

Les cristaux formés par les cellules viables sont piégés dans les filtres polycarbonate servant de support aux cultures épithéliales. Les cultures deviennent uniformément de couleur bleu/pourpre intense lorsqu'elles sont viables, mais restent de couleur blanche/jaune si il y a mortalité cellulaire.

Les résultats sont comparés par rapport aux substances contrôles négatif et positif.

0,15 ml de milieu de culture contenant 10% vol/vol de solution MTT sont pipetés sous chacun des filtres/support de culture. Après une incubation de 30 min à température ambiante, la couleur des différentes cultures est observée.

Les cultures contrôles négatifs doivent être de couleur bleue/pourpre intense, preuve de la viabilité de la muqueuse après 24 heures de contact.

Les cultures contrôles positifs doivent être de couleur blanche, preuve de la mortalité cellulaire dès la 1ère heure de contact.

3- Résultats

Tableau 3

| PRODUIT | Couleur des deux cultures | | | | TOXICITE |
|---|---|---|---|---|---|
| | 10 min | 1 h | 3 h | 24h | |
| BB | bleu | bleu | bleu | blanc | NI |
| Contrôle négatif | bleu | bleu | bleu | bleu | NI |
| SDS 0,5% | bleu | blanc | blanc | / | I |
| SDS 1% | blanc | blanc | / | / | TI |
| NI = non irritant<br>I = irritant<br>TI = très irritant | | | | | |

4- Conclusion

Dans les conditions expérimentales retenues, le produit BB selon l'Exemple 3, étudié pur, est non irritant vis-à-vis des cellules constituant le modèle SKINETHIC® de muqueuse reconstituée *in vitro*.

Exemple 5 : Evaluation de la tolérance cutanée d'un lyophilisat de cellules de gamétophytes d'algues brunes par étude de la cytotoxicité sur épiderme reconstitué SKINETHIC®

1- Protocole expérimental

Des kératinocytes d'origine humaine sont ensemencés sur des filtres en polycarbonate de 0,63 cm$^2$ dans un milieu défini (MCDB 153 modifié) et supplémenté. Les cellules sont cultivées pendant 10 jours à l'interface air/liquide, le milieu de culture étant changé tous les deux jours.

Les épidermes ainsi formés ont été utilisés pour la réalisation de l'étude à partir du 14$^{ème}$ jour de culture.

L'essai a été conduit en triplicate après 24 heures d'incubation du produit, à raison de 2 μl par épiderme. Des épidermes témoins ne reçoivent pas de produit.

Les épidermes fixés dans une solution de formaldéhyde 10 % ont été inclus dans des blocs en paraffine. Les coupes verticales de 4 microns ont été colorées à l'HES (hématoxyline/éosine/safran) et photographiées sous un microscope optique.

Si le produit n'est pas toxique, les cultures doivent présenter des couches cellulaires basales, spineuses, granuleuses et cornées intactes orthokératosique, et la stratification épidermique doit être régulière et normale.

Les cellules de la couche basale doivent être polarisées verticalement. De nombreux grains de kératohyaline doivent être visibles (en violet) dans la couche granuleuse juste sous la couche cornée.

2- Résultats

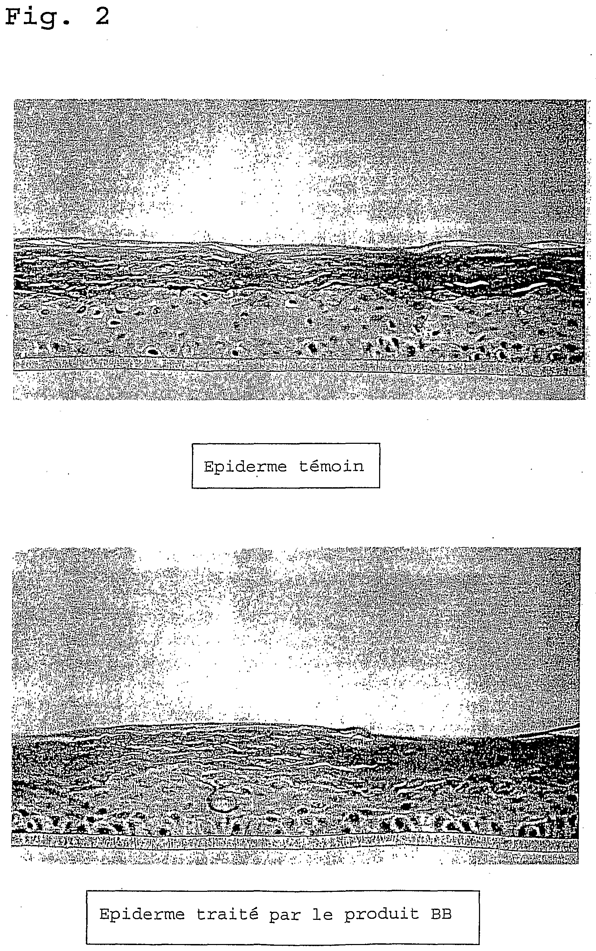

cf. Figure 2

3 - Conclusion

Dans les conditions expérimentales retenues, le produit BB selon l'Exemple 3, étudié pur, s'est révélé non cytotoxique vis-à-vis des épidermes reconstitués SKINETHIC®.

Exemple 6 : Tests de pigmentation

1- Matériel et Méthodes

Le produit BB est dilué dans l'éthanol puis incorporé dans une formulation cosmétique.

Les produits testés sont :

  a- le produit BB incorporé en formulation à 0,05%
  b- le produit BB incorporé en formulation à 0,15%
  c- le produit BB incorporé en formulation à 0,30%

Ils sont conservés à 4°C jusqu'à leur utilisation.

2- Evaluation biochimique

A la fin de l'incubation, la mélanine contenue dans les lysats cellulaires a été quantifiée par une méthode spectrophotométrique. Une gamme de mélanine a été réalisée en parallèle du dosage.

Les résultats sont donnés sous la forme de μg de mélanine par ml de lysat des tapis cellulaires (moyennes +/- la déviation standard SD).

La significativité statistique des différences observées entre les conditions « traitées » et « témoins » a été évaluée par une analyse de la variance à un facteur (One Way ANOVA) suivie d'un test de Bonferroni t-test (* :$p < 0,05$ ;** :$p < 0,01$).

Tableaux 4, 5 et 6 : quantification biochimique du contenu mélanique d'épidermes humains reconstruits mélanisés traités/non traités :

| SANS UV | Milieu seul | α-MSH 100 nM | ETOH BB | | | |
|---|---|---|---|---|---|---|
| | | | 0,05 | 0,15 | 0,3 | 0,15 sans ré-application |
| Mélanine (μg/ml) | 47,3 | 70,3 | 56,6 | 59,9 | 69,1 | 70,3 |
| | 67,3 | 86,9 | 58,7 | 60,5 | 77,2 | 69,0 |
| | 75,8 | 85 | 56,1 | 66,1 | 56 | 58,5 |
| Moyenne | 57,3 | 80,7 | 57,1 | 62,2* | 73,1** | 69,6* |
| S.D. | 14,1 | 9,1 | 1,3 | 3,4 | 5,7 | 0,9 |
| % milieu seul | 100 | 141 | 100 | 108 | 128 | 122 |
| % ETOH BB 0,05 | | | 100 | 108,8 | 128 | 121,9 |

| + UVA | Milieu seul | α-MSH 100 nM | ETOH BB | | | |
|---|---|---|---|---|---|---|
| | | | 0,05 | 0,15 | 0,3 | 0,15 sans ré-application |
| Mélanine (μg/ml) | 57,4 | 95,6 | 69,7 | 63,5 | 64,7 | 61,2 |
| | 73,9 | 84,7 | 66,3 | 64,1 | 66,3 | 66,3 |

| | | 73,4 | 107,9 | 57,3 | 65,7 | | |
|---|---|---|---|---|---|---|---|
| Moyenne | | 68,2 | 96,1 | 64,5 | 64,4 | 65,5 | 62,6 |
| S.D. | | 9,4 | 11,6 | 6,4 | 1,2 | 1,2 | 3,3 |
| % milieu seul | | 100 | 141 | 94 | 94 | 96 | 92 |

| + UVB | Milieu seul | $\alpha$-MSH 100 nM | ETOH BB | | | |
|---|---|---|---|---|---|---|
| | | | 0,05 | 0,15 | 0,3 | 0,15 sans ré-application |
| Mélanine ($\mu$g/ml) | 82,6 | 99,5 | 60,7 | 64,6 | 40,1 | 44,8 |
| | 91,4 | 115,5 | 65,8 | 66,1 | 30,9 | 65,3 |
| | 75,3 | 104,6 | 67,1 | 70,5 | 38,7 | 64,5 |
| Moyenne | 83,1 | 106,5 | 64,5 | 67,1 | 36,6 | 58,2 |
| S.D. | 8 | 8,2 | 3,4 | 3,1 | 5 | 11,6 |
| % milieu seul | 100 | 128 | 78 | 81 | 44 | 70 |

3- Evaluation histologique

Les épidermes ont été fixés dans du formaldéhyde dès la fin de l'incubation, puis inclus dans des blocs de paraffine.

Des coupes de 4 $\mu$m d'épaisseur ont alors été réalisées.

La mélanine présente dans les coupes de peau a été mise en évidence par une coloration spécifique appelée « Fontana Masson ». Dis clichés par condition ont été réalisés au grossissement x400. La mélanine visualisable sur ces clichés a été quantifiée par analyse d'images en utilisant le logiciel ImageJ.

Les résultats sont donnés sous la forme de surface occupée par la mélanine (nombre de pixels dont le niveau de gris est compris entre 0 et 100 ; 0 = noir/255 = blanc) dans chacun des clichés (moyenne +/- la déviation standard, SD).

La significativité statistique des différences observées entre les conditions traitées et les témoins a été évaluée par un test de Student (* : $p<0,05$, ** : $p<0,01$ ; *** : $p<0,001$).

Tableau 7 : quantification du contenu mélanique d'épidermes humains reconstruits mélanisés traités/non traités par analyse d'images:

| SANS UV | Milieu seul | α-MSH100 nM | ETOH BB | | | |
|---|---|---|---|---|---|---|
| | | | 0,05 | 0,15 | 0,3 | 0,15 sans ré-application |
| Surface occupée par la mélanine (pixels) | 24131 | 38565 | 22754 | 29849 | 22821 | 22036 |
| | 17119 | 25757 | 16628 | 15245 | 15368 | 19500 |
| | 30306 | 42233 | 16392 | 36248 | 20144 | 25949 |
| | 13966 | 55615 | 12954 | 7678 | 19722 | 16277 |
| | 9673 | 51363 | 22308 | 6990 | 21260 | 11746 |
| | 19100 | 43652 | 7362 | 10329 | 18916 | 19408 |
| | 13226 | 44188 | 4473 | 10212 | 20203 | 42503 |
| | 20874 | 44053 | 17132 | 22152 | 25844 | 19002 |
| | 26733 | 22738 | 17195 | 25753 | 17680 | 30479 |
| | 18715 | 34614 | 9717 | 15909 | 17895 | 14747 |
| Moyenne | 19384,3 | 40277,8 | 14691,5 | 18036,5 | 19985,3* | 22164,7* |
| S.D. | 6370,9 | 10308,4 | 6034,9 | 10055,1 | 2911,3 | 8944,6 |
| % milieu seul | 100 | 208 | 76 | 93 | 103 | 114 |
| % ETOH BB 0,05 | - | - | 100 | 122,8 | 136 | 150,9 |

3- Résultats

La formulation "ETOH BB 0,15" augmente significativement le contenu en mélanine d'épidermes humains reconstruits mélanisés, non exposés aux UVA ou aux UVB, sans ré-application (+22% par analyse biochimique, +51% par analyse d'images).

La formulation "ETOH BB 0,3" augmente significativement le contenu en mélanine d'épidermes humains reconstruits mélanisés, non exposés aux UVA ou aux UVB (+28% par analyse biochimique, +36% par analyse d'images).

Exemple 7 : effets sur le transport des macromolécules :

7.1- Evaluation de la vitesse du transport des macromolécules (glucides, lipides et protéines) dans un système de microsomes isolés. Effet direct du produit B.B. sur les microsomes isolés :

Les microsomes de kératinocytes humains constituent une fraction membranaire obtenue par centrifugation différentielle à haute vitesse d'un homogénat de cellules.
Cette préparation de microsomes nécessite l'ajout de cofacteurs exogènes tels que le NADPH.

7.1.1-Transport des glucides (glucose)

L'essai a été conduit en triplicate après traitement direct des microsomes.

- Lot 1 : témoin négatif ne recevant aucun produit.
- Lot 2 : témoin positif (phloretine, 0,25 mM)
- Lots 3-5 : traités par le produit à l'étude (3 concentrations)
- Lots 6-8: traités par la phloretine 0,25 mM et par le produit à l'étude (3 concentrations)

Les microsomes obtenus à partir de kératinocytes humains en culture ont été rincés 3 fois avec un tampon PBS sans glucose puis pré-incubés dans 1 ml du même tampon pendant 30 minutes à 37°C. Cette solution a été ensuite éliminée et les microsomes ont été mis dans le tampon PBS sans glucose contenant du 3-0-Méthyl glucose (MG) et du [3H] 3-0-MG sous agitation dans un bain marie à 37°C. La capture du 3-0-MG est arrêtée par l'ajout de 1 ml de PBS froid contenant de la cytochalasine B. La cinétique d'incubation a été réalisée entre 30 et 120 secondes. Les microsomes ont ensuite été rincés 2 fois avec du PBS puis dissous dans NaOH (1M) à 4°C pendant une nuit. La radioactivité a été déterminée à l'aide d'un compteur à scintillation.

Les traitements des microsomes par le produit B.B. seul ou en présence de la phloretine ont été réalisés en même temps que l'introduction du [3H] 3-0-MG dans le milieu d'incubation.

Le dosage des protéines a été réalisé selon la méthode de BRADFORD. L'augmentation de l'absorbance à 595 nm, déterminée à l'aide d'un spectrophotomètre, est proportionnelle à la concentration en protéines.

Résultats :

| | Capture du [$^3$H]3-O-MG (nmol/mg de protéines) | | | |
|---|---|---|---|---|
| | 30 s | 60 s | 90 s | 120 s |
| Témoin négatif | 9 | 15 | 19 | 21 |
| BB 1/10 | 10 | 14 | 20 | 22 |
| BB 1/5 | 9 | 15 | 20 | 22 |
| BB 1/2 | 11 | 16 | 21 | 21 |
| Phloretine (0,25 mM) | 4 | 6 | 8 | 8 |
| BB (1/10) +Phloretine | 5 | 11 | 17 | 19 |
| BB (1/5)+Phloretine | 6 | 12 | 18 | 21 |
| BB (1/2)+Phloretine | 7 | 14 | 18 | 22 |

Les résultats obtenues, après traitement direct des microsomes de kératinocytes par le produit B.B. aux concentrations retenues ne montrent aucune inhibition de la vitesse de transport du glucose. La cinétique de la capture du glucose dans les conditions physiologiques est quasi-identique entre les microsomes témoins et les microsomes traités par le produit B.B. aux 3 concentrations retenues.

Le traitement direct des microsomes par la phloretine inhibe fortement la vitesse de transport du glucose. Le traitement des microsomes par le produit B.B. aux 3 concentrations en même temps que la phloretine rétablit significativement la vitesse du transport du glucose. La cinétique de capture du glucose, dans les conditions d'inhibition est parfaitement rétablie par le produit B.B. aux 3 concentrations retenues.

*7.1.2-Transport des lipides*

L'essai a été conduit en triplicate après traitement direct des microsomes.

- Lot 1 : témoin négatif ne recevant aucun produit.
- Lot 2 : témoin positif (Diphenhydramine 1 mM)
- Lot 3-5 : traité recevant le produit à l'étude (3 concentrations)
- Lot 6-8 : traité par la Diphenhydramine 1 mM et par le produit à l'étude (3 concentrations)

Les microsomes obtenus à partir de kératinocytes humains en culture ont été rincés 3 fois avec un tampon Tris 25 mM puis pré-incubés dans 1 ml du même tampon pendant 30 minutes à 37°C. Cette solution a été ensuite éliminée et les microsomes ont été mis dans le tampon Tris 25 mM contenant du [$^3$H] Choline sous agitation dans un bain marie à 37°C. La capture de la choline a été arrêtée par l'ajout d'un tampon de lyse contenant (50 mM de Tris, 140 mM NaCl, 1,5 mM MgSO4, 0,5% Igepal-Ca-630, 0,2% SDS).La cinétique d'incubation a été réalisée entre 30 et 120 secondes. Les microsomes ont été ensuite rincés 2 fois avec du PBS puis dissous dans NaOH (1M) à 4°C pendant une nuit. La radioactivité a été déterminée à l'aide d'un compteur à Scintillation.

Les traitements des microsomes par le produit B.B. seul ou en présence de la Diphénhydramine (DPA) ont été réalisés en même temps que l'introduction de [$^3$H]Choline dans le milieu d'incubation.

Le dosage des protéines a été réalisé selon la méthode de BRADFORD. L'augmentation de l'absorbance à 595 nm, déterminée à l'aide d'un spectrophotomètre, est proportionnelle à la concentration en protéines.

Résultats :

| | Capture de [$^3$H]choline (pmol/mg de protéines) | | | |
|---|---|---|---|---|
| | 30 s | 60 s | 90 s | 120 s |
| Témoin négatif | 155 | 201 | 261 | 301 |
| BB 1/10 | 175 | 212 | 268 | 310 |
| BB 1/5 | 163 | 210 | 277 | 321 |
| BB 1/2 | 194 | 240 | 289 | 312 |
| DPA (1 mM) | 74 | 105 | 132 | 142 |
| BB (1/10) + DPA | 100 | 121 | 153 | 183 |
| BB (1/5) + DPA | 113 | 131 | 168 | 199 |
| BB (1/2) + DPA | 125 | 140 | 189 | 223 |

Les résultats obtenus, après traitement direct des microsomes de kératinocytes par le produit BB aux concentrations retenues ne montrent aucune inhibition de la vitesse de transport des lipides. La cinétique de la capture de la choline dans les conditions physiologiques est quasi- identique entre les microsomes témoins et les microsomes traités par le produit B.B. aux 3 concentrations retenues.

Les résultats obtenus montrent que le traitement direct des microsomes par la Diphénhydramine inhibe fortement la vitesse de transport des lipides. Le traitement des microsomes par le produit B.B. aux 3 concentrations en même temps que la Diphénhydramine rétablit la vitesse du transport des lipides. La cinétique de capture de la choline, dans les conditions d'inhibition est moyennement rétablie par le produit BB, aux 3 concentrations retenues.

*7.1.3-Transport des protéines (albumine)*

L'essai a été conduit en triplicate après traitement direct des microsomes.

- Lot 1 : témoin négatif ne recevant aucun produit.
- Lot 2 : témoin positif (Nocodazole, 6 mg/ml)
- Lots 3-5 : traités recevant le produit à l'étude (3 concentrations)
- Lots 6-8: traités par le Nocodazole 6 mg/ml et par le produit à l'étude (3 concentrations)

Les microsomes obtenus à partir de kératinocytes humains en culture ont été rincés 3 fois avec un tampon" puis pré-incubés dans 1 ml du même tampon pendant 30 minutes à 3~C. Cette solution a été ensuite éliminée et les microsomes ont été mis dans le tampon HBSS contenant du l'albumine-FITC sous agitation dans un bain marie à 37°C. La capture de l'albumine est arrêtée par l'ajout d'une solution de Ringer contenant (122,5 mM NaCl ; 5,4 mM KCl; 1,2 mM CaCl2; 0,8 mM MgCl2; 0,8 Na2HPO4; 0,2 NaHPO4; 5,5 mM glucose; 10 mM HEPES, pH 7,4). Les microsomes ont été ensuite traités avec du Triton X-100 à (0,1% v/v dans du 3-(N-morpholino) acide propanesulfonique, 20 mM, pH 7,4). La fluorescence a été quantifiée à l'aide d'un spectrophotomètre (excitation 480 nm: émission 520 nm). La cinétique d'incubation a été réalisée entre 30 et 120 secondes.

Les traitements des microsomes par le produit B.B. seul ou en présence de Nocodazole (ND) ont été réalisés en même temps que l'introduction du l'albumine-FITC dans le milieu d'incubation.

Le dosage des protéines a été réalisé selon la méthode de BRADFORD. L'augmentation de l'absorbance à 595 nm est proportionnelle à la concentration des protéines déterminées à l'aide d'un spectrophotomètre.

Résultats :

| | Capture de l'albumine-FITC ($\mu$g/mg de protéines) | | | |
|---|---|---|---|---|
| | 30 s | 60 s | 90 s | 120 s |
| Témoin négatif | 0,32 | 0,45 | 0,55 | 075 |
| BB 1/10 | 0,3 | 0,47 | 0,59 | 0,74 |
| BB 1/5 | 0,37 | 0,5 | 0,62 | 0,76 |
| BB 1/2 | 0,4 | 0,55 | 0,67 | 0,8 |

(suite)

|  | Capture de l'albumine-FITC ($\mu$g/mg de protéines) | | | |
|---|---|---|---|---|
|  | 30 s | 60 s | 90 s | 120 s |
| ND (6 mg/ml) | 0,11 | 0,29 | 0,34 | 0,43 |
| BB (1/10) +ND | 0,12 | 0,3 | 0,33 | 0,41 |
| BB (1/5) +ND | 0,19 | 0,33 | 0,38 | 0,49 |
| BB (1/2) +ND | 0,23 | 0,38 | 0,44 | 0,57 |

Les résultats obtenus, après traitement direct des microsomes de kératinocytes par le produit B.B. aux concentrations retenues ne montrent aucune inhibition de la vitesse de transport des protéines. La cinétique de la capture de l'albumine dans les conditions physiologiques est quasi-identique entre les microsomes témoins et les microsomes traités par le produit B.B. aux 3 concentrations retenues.

Les résultats obtenus montrent que le traitement direct des microsomes par le Nocodazole inhibe fortement la vitesse de transport des lipides. Le traitement des microsomes par le produit B.B. aux concentrations 1/5 et 1/2 en même temps que le Nocodazole rétabli moyennement la vitesse du transport des lipides. Aucun effet n'a été observé à la concentration 1/10.

7.2-Evaluation de la vitesse du transport des macromolécules au niveau des kératinocytes humains normaux

La méthode utilisée a été celle des explants permettant d'obtenir à partir d'une biopsie de peau humaine, des primo-cultures de kératinocytes. Les essais ont été réalisés sur des kératinocytes entre le 2ème et le 4ème passage afin d'assurer une reproductibilité entre les différentes expériences.

Les kératinocytes ont été répartis dans des plaques multipuits (6 puits) à raison de $10^5$ cellules par puits dans 1 ml de milieu de culture SKINETHIC supplémenté par de l'insuline et de l'hydrocortisone. Les cellules ont été incubées en présence et en absence du produit à l'étude.

7.2.1-Transport des glucides (glucose)

L'essai a été conduit en triplicate sur des kératinocytes humains en culture normaux.

- Lot 1 : témoin négatif ne recevant aucun produit.
- Lot 2 : témoin positif (Phloretine, 0,25 mM)
- Lot 3 : traité recevant le produit à l'étude
- Lots 6-8 : traité par la phloretine 0,25 mM et par le produit à l'étude (3 concentrations)

Le traitement des kératinocytes par le produit B.B. en présence et en absence de la phloretine (0,25 mM) a été réalisé pendant 20 minutes à 37 °C. Les microsomes membranaires ont été séparés par centrifugation différentielle.

Le même protocole de capture de [3H] 3-0-Methyl-Glucose a été adopté pour la détermination de la vitesse du transport du glucose. (cf. 6.1.1)

Résultats :

|  | Capture du [$^3$H]3-O-MG (nmol/mg de protéines) | | | |
|---|---|---|---|---|
|  | 30 s | 60 s | 90 s | 120 s |
| Témoin négatif | 6 | 12 | 15 | 19 |
| BB 1/10 | 7 | 10 | 16 | 17 |
| BB 1/5 | 8 | 13 | 17 | 18 |
| BB 1/2 | 10 | 14 | 18 | 20 |
| Phloretine (0,25 mM) | 6 | 8 | 9 | 10 |
| BB (1/10) +Phloretine | 6 | 10 | 15 | 18 |

(suite)

| | Capture du [$^3$H]3-O-MG (nmol/mg de protéines) | | | |
|---|---|---|---|---|
| | 30 s | 60 s | 90 s | 120 s |
| BB (1/5) +Phloretine | 7 | 13 | 16 | 19 |
| BB (1/2) +Phloretine | 8 | 13 | 17 | 18 |

Les résultats obtenus, après traitement des kératinocytes normaux par le produit B.B. préalablement à la séparation des microsomes ne montrent aucune inhibition de la vitesse de transport du glucose. La cinétique de la capture du glucose dans les conditions physiologiques est quasi-identique entre les microsomes des kératinocvtes témoins et les microsomes des kératinocytes traités par le produit B.B. aux 3 concentrations retenues.

Le traitement des kératinocytes normaux par la phloretine préalablement à la séparation des microsomes inhibe fortement la vitesse de transport du glucose. Le traitement des kératinocytes normaux par le produit B.B. aux 3 concentrations en même temps que la phloretine rétabli significativement la vitesse du transport du glucose. La cinétique de capture du glucose, dans les conditions d'inhibition est parfaitement rétablie par le produit B.B. aux 3 concentrations retenues.

*7.2.2-Transport des lipides*

L'essai a été conduit en triplicate sur des kératinocytes humains en culture normaux.

- Lot 1 : témoin négatif ne recevant aucun produit.
- Lot 2 : témoin positif (Diphenhydramine 1 mM)
- Lot 3-5 : traité recevant le produit à l'étude (3 concentrations)
- Lot 6-8: traité par la Diphenhydramine 1 mM et par le produit à l'étude (3 concentrations)

Le traitement des kératinocytes par le produit B.B. en présence et en absence de la Diphenhydramine 1 mM a été réalisé pendant 120 minutes à 37 °C. Les microsomes membranaires ont été séparés par centrifugation différentielle.

Le même protocole de capture de [3H] Choline a été adopté pour la détermination de la vitesse du transport de la choline (cf. 6.1.2)

Résultats :

| | Capture de [$^3$H]choline (pmol/mg de protéines) | | | |
|---|---|---|---|---|
| | 30 s | 60 s | 90 s | 120 s |
| Témoin négatif | 142 | 173 | 202 | 215 |
| BB 1/10 | 151 | 163 | 198 | 223 |
| BB 1/5 | 162 | 178 | 207 | 227 |
| BB 1/2 | 174 | 191 | 218 | 231 |
| DPA (1 mM) | 92 | 115 | 143 | 156 |
| BB (1/10> + DPA | 112 | 128 | 159 | 174 |
| BB (1/5) + DPA | 119 | 132 | 165 | 179 |
| BB (1/2) + DPA | 127 | 139 | 174 | 191 |

Les résultats obtenus, après traitement des kératinocytes normaux par le produit B.B. préalablement à la séparation des microsomes ne montrent aucune inhibition de la vitesse de transport des lipides. La cinétique de la capture de la choline dans les conditions physiologiques est quasi-identique entre les microsomes des kératinocytes témoins et les microsomes des kératinocytes traités par le produit B.B. aux 3 concentrations retenues.

Le traitement des kératinocytes normaux par la Diphenhydramine préalablement à la séparation des microsomes inhibe modérément la vitesse de transport des lipides. Le traitement des kératinocytes normaux par le produit BB aux 3 concentrations en même temps que la Diphenhydramine rétablit la vitesse du transport des lipides. La cinétique de capture de la choline, dans les conditions d'inhibition est moyennement rétablie par le produit BB aux 3 concentrations retenues.

*7.2.3- Transport des protéines (albumine)*

L'essai a été conduit en triplicate sur des kératinocytes humains en culture normaux.

- Lot 1 : témoin négatif ne recevant aucun produit.
- Lot 2 : témoin positif (Nocodazole, 6 mg/ml)
- Lot 3-5 : traité recevant le produit à l'étude (3 concentrations)
- Lot 6-8 : traité par la Nocodazole, 6 mg/ml et par le produit à l'étude (3 concentrations)

Le traitement des kératinocytes par le produit B.B. en présence et en absence du Nocodazole, 6 mg/ml a été réalisé pendant 120 minutes à 37 °C. Les microsomes membranaires ont été séparés par centrifugation différentielle.

Le même protocole de capture de l'albumine-FITC a été adopté pour la détermination de la vitesse du transport de l'albumine (cf. 3.1.4.2)

Résultats :

|  | Capture de l'albumine-FITC ($\mu$g/mg de protéines) | | | |
|---|---|---|---|---|
|  | 30 s | 60 s | 90 s | 120 s |
| Témoin négatif | 0,25 | 0,39 | 0,45 | 0, 61 |
| BB 1/10 | 0,24 | 0,40 | 0,48 | 0,63 |
| BB 1/5 | 0,28 | 0,44 | 0,52 | 0,67 |
| BB 1/2 | 0,31 | 0,46 | 0,54 | 0,73 |
| ND (6 mg/ml) | 0,09 | 0,22 | 0,26 | 0,37 |
| BB (1/10) +ND | 0,1 | 0,24 | 0,26 | 0,39 |
| BB (1/5) +ND | 0,12 | 0,27 | 0,30 | 0,41 |
| BB (1/2) +ND | 0,16 | 0,29 | 0,36 | 0,47 |

Les résultats obtenus, après traitement des kératinocytes normaux par le produit B.B. préalablement à la séparation des microsomes ne montrent aucune inhibition de la vitesse de transport des protéines. La cinétique de la capture de l'albumine dans les conditions physiologiques est identique entre les microsomes des kératinocytes témoins et les microsomes des kératinocytes traités par le produit B.B. à la concentration 1/10. Une faible augmentation de la vitesse du transport des protéines est observée aux concentrations 1/2 et 1/5.

Le traitement des kératinocytes normaux par le Nocodazole préalablement à la séparation des microsomes inhibe la vitesse de transport des protéines. Le traitement des kératinocytes normaux par le produit B.B. aux 2 concentrations en même temps que le Nocodazole rétablit moyennement la vitesse du transport des protéines. Aucun effet n'a été observé à la concentration 1/10.

7.3- Evaluation de la vitesse du transport des macromolécules au niveau des kératinocytes humains en sénescence

La sénescence est un phénomène de vieillissement cellulaire, où les cellules sont bloquées en phase G1 du cycle cellulaire et ne rentrent jamais dans la phase de synthèse conduisant à la division cellulaire. Les cellules sénescentes sont caractérisées par leur métabolisme lent au niveau de la synthèse et du transport des macromolécules.

Les cellules en culture privées de sérum, arrêtent de croître, mais continuent de traverser le cycle cellulaire jusqu'à ce qu'elles atteignent la phase G1.

La méthode utilisée a été celle permettant d'obtenir, à partir d'une biopsie de peau humaine, des primo-cultures de kératinocytes. Les essais ont été réalisés sur des kératinocytes, entre le 8ème et le 10ème passage, afin d'assurer la présence des cellules sénescentes au niveau des cellules témoins.

Les kératinocytes ont été ensemencés dans des plaques multipuits (6 puits), à raison de $10^5$ cellules par puit dans 3 ml de milieu de culture SKINETHIC supplémenté par l'EGF, l'hydrocortisone, l'insuline et la gentamicyne. Ils ont été ensuite maintenus pendant 5 jours en étuve à $CO_2$.

*7.3.1-Transport des glucides (glucose)*

L'essai a été conduit en triplicate sur des kératinocytes humains en sénescence.

- Lot 1 : témoin négatif ne recevant aucun produit.
- Lot 2 : témoin positif (Phloretine, 0,25 mM)
- Lot 3 : traité recevant le produit à l'étude
- Lots 6-8 : traités par la phloretine 0,25 mM et par le produit à l'étude (3 concentrations)_

Le traitement des kératinocytes par le produit B.B. en présence et en absence de la phloretine (0,25 mM) a été réalisé pendant 20 minutes à 37°C. Les microsomes membranaires ont été séparés par centrifugation différentielle.
Le même protocole de capture de [3H] 3-0-Methyl-Glucose a été adopté pour la détermination de la vitesse du transport du glucose (cf. 6.1.1).

Résultats :

| | Capture du $[^3H]$3-O-MG (nmol/mg de protéines) | | | |
|---|---|---|---|---|
| | 30 s | 60 s | 90 s | 120 s |
| Témoin négatif | 3 | 5 | 10 | 11 |
| BB 1/10 | 4 | 8 | 12 | 12 |
| BB 1/5 | 6 | 8 | 13 | 13 |
| BB 1/2 | 7 | 10 | 14 | 15 |
| Phloretine (0,25 mM) | 1 | 3 | 5 | 7 |
| BB (1/10) +Phloretine | 3 | 5 | 7 | 8 |
| BB (1/5) +Phloretine | 5 | 8 | 9 | 11 |
| BB (1/2) +Phloretine | 6 | 10 | 12 | 13 |

Les résultats obtenus montrent que la vitesse de transport du glucose est nettement faible aux niveaux des kératinocytes en sénescence par rapport aux kératinocytes normaux.
Le traitement des kératinocytes en sénescence par le produit B.B., préalablement à la séparation des microsomes, montre une augmentation de la vitesse de transport du glucose par rapport aux cellules témoins non traitées. La cinétique de la capture du glucose dans les conditions physiologiques est supérieure au niveau des microsomes des kératinocytes traités par le produit B.B. par rapport aux microsomes des kératinocytes témoins. Ce résultat est obtenu aux différents temps d'incubations (30, 60, 90 et 120 secondes).
Le traitement des kératinocytes en sénescence par la phloretine préalablement à la séparation des microsomes inhibe fortement la vitesse de transport du glucose. Le traitement des kératinocytes normaux par le produit B.B. aux 2 concentrations (1/5 et 1/2) en même temps que la phloretine rétablit significativement la vitesse du transport du glucose. Un faible effet est observé avec la concentration 1/10.

*7.3.2- Transport des lipides*

L'essai a été conduit en triplicate sur des kératinocytes humains en sénescence.

- Lot 1 : témoin négatif ne recevant aucun produit.
- Lot 2 : témoin positif (Diphenhydramine 1 mM)
- Lot 3-5 : traité recevant le produit à l'étude (3 concentrations)
- Lot 6-8: traité par la Diphenhydramine 1 mM et par le produit à l'étude (3 concentrations)

Le traitement des kératinocytes par le produit B.B. en présence et en absence de la Diphenhydramine 1 mM a été réalisé pendant 120 minutes à 37 °C. Les microsomes membranaires ont été séparés par centrifugation différentielle.
Le même protocole de capture de [3H] Choline a été adopté pour la détermination de la vitesse du transport de la choline (cf. 6.1.2)

Résultats :

| | Capture de $[^3H]$ choline (pmol/mg de protéines) | | | |
|---|---|---|---|---|
| | 30 s | 60 s | 90 s | 120 s |
| Témoin négatif | 114 | 132 | 164 | 181 |
| BB 1/10 | 110 | 135 | 166 | 173 |
| BB 1/5 | 113 | 143 | 188 | 194 |
| BB 1/2 | 121 | 153 | 187 | 201 |
| DPA (1 mM) | 55 | 79 | 106 | 119 |
| BB (1/10) + DPA | 52 | 69 | 110 | 125 |
| BB (1/5) + DPA | 67 | 87 | 132 | 141 |
| BB (1/2) + DPA | 77 | 104 | 137 | 148 |

Les résultats obtenus, montrent que la vitesse de transport des lipides est nettement faible au niveau des kératinocytes en sénescence par rapport aux kératinocytes normaux.

Le traitement des kératinocytes en sénescence par le produit B.B., préalablement à la séparation des microsomes, ne montre aucune inhibition de la vitesse de transport des lipides par rapport aux cellules témoins non traitées. La cinétique de la capture des lipides dans les conditions physiologiques est comparable entre les microsomes des kératinocytes traités par le produit B.B. et les microsomes des kératinocytes témoins.

Le traitement des kératinocytes en sénescence par la Diphenhydramine, préalablement à la séparation des microsomes, inhibe fortement la vitesse de transport des lipides. Le traitement des kératinocytes en sénescence par le produit B.B. aux 2 concentrations (1/5 et 1/2) en même temps que la Diphenhydramine rétablit significativement la vitesse du transport des lipides. Aucun effet n'a été observé avec la concentration 1/10.

*7.3.3-Transport des protéines (albumine)*

L'essai a été conduit en triplicate sur des kératinocytes humains en sénescence.

- Lot 1 : témoin négatif ne recevant aucun produit.
- Lot 2 : témoin positif (Nocodazole, 6 mg/ml)
- Lot 3-5 : traité recevant le produit à l'étude (3 concentrations)
- Lot 6-8 : traité par la Nocodazole, 6 mg/ml et par le produit à l'étude (3 concentrations)

Le traitement des kératinocytes par le produit B.B. en présence et en absence de Nocodazole, 6 mg/ml a été réalisé pendant 120 minutes à 37 °C. Les microsomes membranaires ont été séparés par centrifugation différentielle.

Le même protocole de capture de l'albumine-FITC a été adopté pour la détermination de la vitesse du transport de l'albumine (cf. 6.1.3)

Résultats :

| | Capture de l'albumine-FITC ($\mu$g/mg de protéines) | | | |
|---|---|---|---|---|
| | 30 s | 60 s | 90 s | 120 s |
| Témoin négatif | 0,17 | 0,29 | 0,35 | 0,49 |
| BB 1/10 | 0,18 | 0,34 | 0,39 | 0,52 |
| BB 1/5 | 0,18 | 0,36 | 0,41 | 0,54 |
| BB 1/2 | 0,2 | 0,37 | 0,43 | 0,55 |
| ND (6 mg/ml) | 0,06 | 0,11 | 0,19 | 0,3 |
| BB (1/10) +ND | 0,05 | 0,12 | 0,22 | 0,33 |
| BB (1/5) +ND | 0,09 | 0,14 | 0,27 | 0,38 |

(suite)

|  | Capture de l'albumine-FITC ($\mu$g/mg de protéines) | | | |
|---|---|---|---|---|
|  | 30 s | 60 s | 90 s | 120 s |
| BB (1/2) +ND | 0,1 | 0,19 | 0,3 | 0,43 |

Les résultats obtenus, montrent que la vitesse de transport des protéines est nettement faible aux niveaux des kératinocytes en sénescence par rapport aux kératinocytes. normaux. Le traitement des kératinocytes en sénescence par le produit B.B. préalablement à la séparation des microsomes ne montre aucune inhibition de la vitesse de transport des protéines par rapport aux cellules témoins non traitées. La cinétique de la capture de l'albumine dans les conditions physiologiques est comparable entre les microsomes des kératinocytes traités par le produit B.B. et les microsomes des kératinocytes témoins.

Le traitement des kératinocytes en sénescence par le Nocodazole préalablement à la séparation des microsomes inhibe la vitesse de transport des protéines. Le traitement des kératinocytes en sénescence par le produit B.B. aux 2 concentrations (1/5 et 1/2), en même temps que le Nocodazole, rétablit significativement la vitesse du transport des protéines. Un faible effet est observé avec la concentration 1/10.

Exemple 8 : effet sur la respiration et la synthèse d'ATP 8.1-Etude de l'effet du produit sur la respiration cellulaire et mitochondriale

La quantité d'oxygène dissout dans une solution peut être déterminée à l'aide d'une électrode de Clark. L'oxygène diffusant à travers un film de téflon va être réduit au niveau de la cathode de platine polarisée à -0.8 Volt. Dans ces conditions, le courant passant entre cette cathode et l'anode d'argent est proportionnel à la concentration en oxygène dans la solution. Le pont ionique entre les deux électrodes est assuré par une solution de KCl saturée.

L'acquisition et le traitement des mesures sont faits sur un micro-ordinateur (IBM-PC) en temps réel. Un programme permet de visualiser en continu la quantité d'oxygène dans la cuve ainsi que la dérivée instantanée correspondant à la vitesse de consommation d'oxygène qui est calculée en temps réel.

Cinq concentrations ont été testées et les déterminations ont été effectuées en triplicate. Cette étude est réalisée selon deux conditions différentes:

*8.1.1 Effet du produit sur la respiration cellulaire*: *Mesure de la respiration basale*

Effet sur la vitesse de respiration basale sur cellules non perméabilisées en présence de glucose. Les kératinocytes ont été cultivés, en étuve à $CO_2$, à raison de $10^6$ par run dans un milieu de culture DMEM supplémenté par l'hydrocortisone, l'EGF et le SVF (10%).

Ce protocole a été réalisé par application directe du produit sur les cellules dans la cuve de l'oxygraphe.

Les cellules (kératinocytes), à la concentration de 106 cellules/ml, sont mises en suspension dans un "tampon respiratoire" (Hanks-Hepes glucose 20 mM), dans la cuve de l'oxygraphe thermostatée à 30°C et équipée d'une électrode de Clark (1 ml de tampon respiratoire contenant dans ces conditions 480 atomes d'oxygène).

Dans ces conditions, une vitesse de consommation d'oxygène (respiration basale des cellules) peut être mesurée.

L'ajout de différentes quantités du produit (concentrations finales: 1/10; 1/5 et 1/2) dans la cuve de l'oxygraphe permet de mettre en évidence une éventuelle stimulation ou inhibition de cette respiration.

L'ensemble des déterminations est résumé dans le tableau ci-dessous.

| Produit testé (BB) | Contrôle | 1/10 | 1/5 | 1/2 |
|---|---|---|---|---|
| Vitesse de respiration (nAtom O/min/$10^6$ cellules) n=3 | 3,64 $\pm$ 0,37 | 3,72 $\pm$ 0,31 | 4,26 $\pm$ 0,25 | 4,75 $\pm$ 0,50 |
| % de respiration basale n=3 | 100 $\pm$ 6 | 102 $\pm$ 4 | 117 $\pm$ 3 | 130 $\pm$ 5 |

Dans les conditions expérimentales retenues et au vu des résultats obtenus, le produit au contact des kératinocytes induit une augmentation significative de la vitesse de respiration basale aux concentrations (1/5 et 1/2).

*8.1.2. Effet du produit sur la respiration mitochondriale :*

Effet sur la vitesse de la respiration des cellules perméabilisées en présence du substrat respiratoire, pyruvate-malate, pour évaluer la respiration mitochondriale.

Les kératinocytes ont été cultivés, en étuve à $CO_2$, à raison de $10^6$ cellules par run dans un milieu de culture DMEM supplémenté par l'hydrocortisone, l'EGF et le SVF (10%).

Ce protocole a été réalisé par application directe du produit sur les cellules dans la cuve de l'oxygraphe.

Les cellules (kératinocytes), à la concentration de $10^6$ cellules/ml, sont mises en suspension dans un "tampon respiratoire" (Hanks-Hepes glucose 20 mM), dans la cuve de l'oxygraphe thermostatée à 30°C et équipée d'une électrode de Clark (1 ml de tampon respiratoire contenant dans ces conditions 480 atomes d'oxygène). Les cellules sont perméabilisées à l'aide de digitonine. L'ajout d'un substrat respiratoire (pyruvate 10 mM et malate 10 mM) permet d'observer une vitesse de consommation d'oxygène (état 2 selon Chance). L'ajout de différentes quantités du produit (concentrations finales: 1/10; 1/5 et 1/2) dans la cuve de l'oxygraphe permet de mettre en évidence une éventuelle stimulation ou inhibition de cette respiration.

L'ensemble des déterminations est résumé dans le tableau ci-dessous.

| Produit testé (BB) | Contrôle | 1/10 | 1/5 | 1/2 |
|---|---|---|---|---|
| Vitesse de respiration (nAtom O/min/$10^6$ cellules) n=3 | 2, 67 $\pm$ 0,21 | 2,83 $\pm$ 0,15 | 3,25 $\pm$ 0,11 | 3,52 $\pm$ 0,09 |
| % de respiration basale n=3 | 100 $\pm$ 2 | 106 $\pm$ 4 | 122 $\pm$ 3 | 132 $\pm$ 5 |

Dans les conditions expérimentales retenues et au vu des résultats obtenus, le produit au contact des kératinocytes humains en culture induit une augmentation significative de la vitesse de respiration mitochondriale aux concentrations (1/5 et 1/2).

8.2-Etude de l'effet du produit sur la synthèse d'ATP mitochondriale

La mesure s'effectue à l'aide d'un appareil de type Luminoscan en utilisant l'ATP monitoring reagent (ATP Bioluminescence Assay Kit HS II) de Boehringer Mannheim. La quantité d'ATP présente dans cet aliquot peut être déterminée grâce à la réaction enzymatique suivante:

$$ATP + LUCIFERINE \xrightarrow[Mg2+]{Luciférase} OXYLUCIFERINE + AMP + PPi + CO_2$$

L'intensité de la lumière émise lors de cette réaction peut être mesurée par un luminomètre (Luminoscan) qui la transcrit en RLU (unité relative de luminosité). Les RLU mesurées peuvent être converties en moles d'ATP en se référant à une gamme étalon d'ATP.

La vitesse de synthèse d'ATP est exprimée en nmoles/min/$10^6$ cellules.

Cinq concentrations ont été testées et les déterminations ont été effectuées en triplicate.

Les kératinocytes ont été cultivés, en étuve à $CO_2$, à raison de $10^6$ par run dans un milieu de culture DMEM supplémenté par l'hydrocortisone, l'EGF et le SVF (10%).

Ce protocole a été réalisé par application directe du produit sur les cellules dans la cuve de l'oxygraphe.

Les cellules (kératinocytes), à la concentration de $10^6$ cellules/ml, sont mises en suspension dans un "tampon respiratoire" (Hanks-Hepes glucose 20 mM), dans la cuve de l'oxygraphe thermostatée à 30°C. Les cellules sont perméabilisées à l'aide de digitonine. L'ajout d'un substrat respiratoire (pyruvate 10 mM et malate 10 mM) permet d'observer une vitesse de consommation d'oxygène (état 2 selon Chance). Après l'ajout de différentes quantités du produit (concentrations: 1/10 ; 1/5 et 1/2) dans la cuve de l'oxygraphe et à intervalles réguliers, un aliquot est prélevé dans la cuve de l'oxygraphe pour y doser l'ATP selon la méthode décrite ci-dessus. L'addition de différentes quantités du produit dans la cuve de l'oxygraphe permet ainsi de mettre en évidence une éventuelle activation ou inhibition de la synthèse d'ATP.

L'ensemble des déterminations est résumé dans le tableau ci-dessous.

| Produit testé (BB) | Contrôle | 1/10 | 1/5 | 1/2 |
|---|---|---|---|---|
| Vitesse de synthèse d'ATP (nmol/min/$10^6$ cellules) n=3 | 5,78 $\pm$ 0,25 | 6,16 $\pm$ 0,34 | 6,96 $\pm$ 0,29 | 7,24 $\pm$ 0,54 |
| % de synthèse d'ATP n=3 | 100 $\pm$ 3 | 107 $\pm$ 5 | 120 $\pm$ 8 | 125 $\pm$ 6 |

Dans les conditions expérimentales retenues et au vu des résultats obtenus, le produit au contact des kératinocytes humains en culture induit une augmentation significative de la vitesse de synthèse d'ATP mitochondriale aux concentrations (1/5 et 1/2).

8.3-Etude de l'effet du produit sur le métabolisme énergétique

Evaluation de l'effet du produit au niveau du métabolisme énergétique par mesure des concentrations des nucléotides adényliques. Etude réalisée sur des primo-cultures de kératinocytes humaines traitées pendant 5 jours avant l'étude.

La quantité d'ATP, ADP et AMP contenue dans les cellules est mesurée par la technique de Chromatographie Liquide à Haute Pression (HPLC).

Sur un appareil Beckman, nous utilisons une colonne (10 x 0,46 cm) dont le support est le Sphérisorb NH$_2$ de 5 $\mu$m de granulométrie. Le solvant d'élution est une solution de phosphate de potassium ; suivant la molarité et le pH de celui-ci, le temps de rétention des nucléotides est plus ou moins long. La vitesse d'élution est de 1 ml/min. Le profil d'élution est suivi par mesure d'absorbance à 254 nm en socratique. Une gamme étalon est réalisée entre 0,1 et 1 nmole d'ATP, d'ADP et d'AMP en mesurant la surface de l'aire sous les pics respectifs.

Cinq concentrations ont été testées et les déterminations ont été effectuées en triplicate.

Les concentrations d'ATP, ADP et AMP d'un extrait cellulaire ($10^6$ cellules/ml) sont dosées par HPLC. Elles sont exprimées en nmoles/min/mg protéines. La charge énergétique est calculée par la formule:

$$([ATP] + 1/2[ADP])/([ATP]+[ADP]+[AMP])$$

Le calcul de la charge énergétique est effectué en triplicate sur des lots témoins et des lots traités.

L'ensemble des déterminations est résumé dans le tableau ci-dessous.

Les concentrations en ATP, ADP et AMP sont exprimées en nmoles/mg protéines (n=3).

| Produit testé BB | Contrôle | 1/10 | 1/5 | 1/2 |
|---|---|---|---|---|
| [ATP] | 4214 | 4060 | 4554 | 4860 |
| [ADP] | 936 | 1103 | 1162 | 936 |
| [AMP] | 673 | 488 | 719 | 1178 |
| [ATP/ADP] | 4,50 | 3,68 | 3,92 | 5,19 |
| Somme | 5823 | 5651 | 6434 | 6973 |
| C.E. | 0,804 | 0,816 | 0,798 | 0,764 |

Dans les conditions expérimentales retenues et au vu des résultats obtenus, le produit au contact des kératinocytes humains en culture n'induit aucune modification de la charge énergétique aux concentrations du produit utilisées.

**Revendications**

1.   Lyophilisat de gamétophytes d'algues brunes.

2.   Procédé d'obtention d'un lyophilisat selon la revendication 1, **caractérisé en ce qu'**il comprend les étapes suivantes :

- récolte des sporophytes matures
- émission des spores in vitro
- germination des spores in vitro
- récolte des cellules de gamétophytes
- lyophilisation des cellules de gamétophytes obtenues.

3.   Préparation cosmétique à usage topique **caractérisée en ce qu'**elle comprend un lyophilisat selon la revendication 1 en tant que principe actif.

4. Préparation cosmétique selon la revendication 3, ledit lyophilisat se trouvant dans la gamme de 0, 2 à 5% en poids.

5. Complément alimentaire **caractérisé en ce qu'**il comprend un lyophilisat selon la revendication 1 en tant que principe actif.

6. Préparation à usage topique comprenant un lyophilisat selon la revendication 1 en tant que principe actif, pour son utilisation pour pigmenter la peau dans une méthode de traitement thérapeutique du corps humain.

7. Préparation à usage topique comprenant un lyophilisat selon la revendication 1 en tant que principe actif, pour son utilisation pour préparer la peau à une exposition aux rayons UV, dans une méthode de traitement thérapeutique du corps humain.

8. Préparation à usage topique comprenant un lyophilisat selon la revendication 1 en tant que principe actif, pour son utilisation pour protéger la peau du stress oxydatif dans une méthode de traitement thérapeutique du corps humain.

9. Préparation à usage topique comprenant un lyophilisat selon la revendication 1 en tant que principe actif, pour son utilisation pour protéger la peau contre le vieillissement cellulaire dans une méthode de traitement thérapeutique du corps humain.

**Patentansprüche**

1. Lyophilisat von Braunalgen-Gametophyten.

2. Verfahren zur Gewinnung eines Lyophilisats nach Anspruch 1, **dadurch gekennzeichnet, dass** es die folgenden Schritte umfasst:

   - Ernte der reifen Sporophyten
   - Freisetzung der Sporen in vitro
   - Keimung der Sporen in vitro
   - Ernte der Gametophyten-Zellen
   - Lyophilisierung der gewonnenen Gametophyten-Zellen.

3. Kosmetische Zubereitung zur topischen Anwendung, **dadurch gekennzeichnet, dass** sie ein Lyophilisat nach Anspruch 1 als Wirkstoff umfasst.

4. Kosmetische Zubereitung nach Anspruch 3, wobei sich das Lyophilisat im Bereich von 0, 2 bis 5 Gewichts-% befindet.

5. Nahrungsergänzungsmittel, **dadurch gekennzeichnet, dass** es ein Lyophilisat nach Anspruch 1 als Wirkstoff umfasst.

6. Zubereitung zur topischen Anwendung, welche ein Lyophilisat nach Anspruch 1 als Wirkstoff umfasst, zur ihrer Verwendung in einem Verfahren zur therapeutischen Behandlung des menschlichen Körpers, um die Haut zu pigmentieren.

7. Zubereitung zur topischen Anwendung, welche ein Lyophilisat nach Anspruch 1 als Wirkstoff umfasst, zur ihrer Verwendung in einem Verfahren zur therapeutischen Behandlung des menschlichen Körpers, um die Haut auf eine Exposition gegenüber UV-Strahlen vorzubereiten.

8. Zubereitung zur topischen Anwendung, welche ein Lyophilisat nach Anspruch 1 als Wirkstoff umfasst, zur ihrer Verwendung in einem Verfahren zur therapeutischen Behandlung des menschlichen Körpers, um die Haut vor oxidativem Stress zu schützen.

9. Zubereitung zur topischen Anwendung, welche ein Lyophilisat nach Anspruch 1 als Wirkstoff umfasst, zur ihrer Verwendung in einem Verfahren zur therapeutischen Behandlung des menschlichen Körpers, um die Haut vor Zellalterung zu schützen.

**Claims**

1. Lyophilisate of gametophytes of brown algae.

2. Method for obtaining a lyophilisate according to claim 1, **characterised in that** said method comprises the following steps:

   - harvesting the mature sporophytes
   - emitting the spores *in vitro*
   - germinating the spores *in vitro*
   - harvesting the gametophyte cells
   - lyophilisation of the gametophyte cells obtained.

3. Cosmetic preparation for topical use, **characterised in that** said preparation comprises a lyophilisate according to claim 1 as an active principle.

4. Cosmetic preparation according to claim 3, said lyophilisate being in the range of 0, 2 to 5% by weight.

5. Food supplement **characterised in that** said supplement comprises a lyophilisate according to claim 1 as an active principle.

6. Preparation for topical use, comprising a lyophilisate according to claim 1 as an active principle, for the use thereof to pigment the skin in a method for therapeutic treatment of the human body.

7. Preparation for topical use, comprising a lyophilisate according to claim 1 as an active principle, for the use thereof to prepare the skin for exposure to UV rays in a method for therapeutic treatment of the human body.

8. Preparation for topical use, comprising a lyophilisate according to claim 1 as an active principle, for the use thereof to protect the skin from oxidative stress in a method for therapeutic treatment of the human body.

9. Preparation for topical use, comprising a lyophilisate according to claim 1 as an active principle, for the use thereof to protect the skin against cell aging in a method for therapeutic treatment of the human body.

# Fig. 1

# Fig. 2

Epiderme témoin

Epiderme traité par le produit BB

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6433025 B
- GB 1323800 A